# EUROPEAN PATENT APPLICATION

(11) **EP 3 943 034 A1**
(43) Date of publication of application: **26.01.2022**
(21) Application number: 20911917.1
(22) Date of filing: 09.01.2020
(51) Int. Cl.: A61B 34/30

(54) **SURGICAL TOOL**

(71) Applicant: Riverfield Inc., Shinjuku-ku Tokyo 160-0017 (JP)
(72) Inventor: SHINDO, Koki, Tokyo 160-0017 (JP)
(74) Representative: adares Patent- und Rechtsanwälte Reininger & Partner GmbB
(86) International application number: PCT/JP2020/000499
(87) International publication number: WO 2021/140626

(57) **Abstract**

There is provided a surgical tool. The surgical tool of the present disclosure includes one or more driven portions to transmit a driving force to a distal end of a shaft to be inserted into a patient's body; a main body holding the one or more driven portions so as to be relatively movable in respective specified directions; and one or more fixing portions each configured to move in a direction intersecting a corresponding one of the specified directions and extending toward a corresponding one of the one or more driven portions and the main body, and to be abuttable with the corresponding one of the one or more driven portions and with the main body.

## Description

### TECHNICAL FIELD

The present disclosure relates to a surgical tool.

### BACKGROUND ART

There is a possibility that while a medical treatment, such as an operation using a medical robot, is performed, power supply to the medical robot is stopped, for example, due to emergency stop. It is required to prevent a treatment portion in a surgical tool provided to the medical robot from making a movement unintended by an operator while power supply is stopped. Examples of the surgical tool may include a forceps, and examples of the treatment portion may include a holding portion.

As a technique of preventing unintended movement of the treatment portion, there is proposed a technique, for example, in which a rotation locking mechanism is provided to a drive portion, to thereby prevent a movable portion of the surgical tool from moving in an unnecessary direction (see Patent Document 1).

### PRIOR ART DOCUMENTS

### PATENT DOCUMENTS

Patent Document 1: JP2019521786A

### SUMMARY OF THE INVENTION

### PROBLEMS TO BE SOLVED BY THE INVENTION

A driven interface of a driven disk in an instrument disclosed in Patent Document 1 includes an engagement receptacle, a drive dog receptacle, and a rotation disable element. The rotation disable element includes a rotation locking mechanism. In case of disabling rotation, the rotation locking mechanism is engaged with a driven disk receptacle. As a result, the driven disk is prevented from rotating, and rotation is disabled.

However, the configuration to disable rotation in Patent Document 1 includes components, such as the engagement receptacle, the drive dog receptacle, and the rotation disable element. These components involve a problem that their configurations require time and labor for cleaning, for example, because of their complicated shapes.

It is desirable that the present disclosure provides a surgical tool that enables reducing unintended movement of the surgical tool and also facilitates cleaning of the surgical tool.

### MEANS FOR SOLVING THE PROBLEMS

A surgical tool of the present disclosure comprises one or more driven portions to transmit a driving force to a distal end of a shaft to be inserted into a patient's body; a main body holding the one or more driven portions so as to be relatively movable in respective specified directions; and one or more fixing portions each configured to move in a direction intersecting a corresponding one of the specified directions and extending toward a corresponding one of the one or more driven portions and the main body, and to be abuttable with the corresponding one of the one or more driven portions and with the main body. Hereinafter, these may also be referred to simply as a driven portion and a fixing portion.

According to this configuration, the fixing portion that has moved is pressed against the driven portion and the main body. The driven portion pressed against the fixing portion is also pressed against the main body. A friction force as a force resisting relative movement between the driven portion and the main body is generated in an abutting area between the fixing portion and the driven portion, and in an abutting area between the driven portion and the main body. This facilitates reduction of unintended movement of the surgical tool when moving the fixing portion toward the driven portion and the main body.

Also, the number of components is reduced as compared with the configuration described in Patent Document 1. Further, it is easy to achieve simplified configurations of those components. Accordingly, it is easy to reduce work required for cleaning, and it is easy to reduce time required for cleaning.

In the disclosure above, the driven portion and/or the main body may preferably comprise one or more first inclined surfaces to guide the fixing portion to a gap between the driven portion and the main body.

With this configuration, the fixing portion is easily guided to the gap between the driven portion and the main body. The fixing portion guided to the gap is pressed against the driven portion and the main body. This facilitates reduction of unintended movement of the surgical tool when moving the fixing portion toward the gap between the driven portion and the main body.

In the disclosure above, one or more second inclined surfaces may be preferably provided to the fixing portion in positions to face the first inclined surfaces.

With this configuration, the fixing portion is more easily guided to the gap between the driven portion and the main body. This facilitates further reduction of unintended movement of the surgical tool when moving the fixing portion toward the gap between the driven portion and the main body.

Also, since the second inclined surface abuts the first inclined surface, an area of mutual abutment can be easily secured. In an abutting area between the first inclined surface and the second inclined surface, a friction force as a force resisting the relative movement between the driven portion and the main body is generated. This facilitates further reduction of unintended movement of the surgical tool when moving the fixing portion toward the gap between the driven portion and the main body.

In the disclosure above, it may be preferable that two or more driven portions are provided and arranged aligned with each other on a plane that includes the specified directions and does not include the intersecting directions, and that each of the one or more fixing portions is abuttable with a corresponding one of a part of the two or more driven portions and with the main body.

With this configuration, movement of the surgical tool to be restricted and movement of the surgical tool to be not restricted can be differentiated. Specifically, it is configured such that the fixing portion is abuttable with the driven portion related to the movement of the surgical tool to be restricted and with the main body, while the fixing portion is not abuttable with the driven portion related to the movement of the surgical tool to be not restricted or with the main body, thereby enabling differentiation between the movement of the surgical tool to be restricted and the movement of the surgical tool to be not restricted.

In the disclosure above, the shaft may preferably comprise a joint portion allowing changes in orientation of the distal end of the shaft by the driving force transmitted from the driven portions, and each of the one or more fixing portions is abuttable with the corresponding one of the one or more driven portions to transmit the driving force to the joint portion and with the main body.

With this configuration, movement of the joint portion can be restricted. It is possible to reduce changes in orientation of the distal end of the shaft that is inserted in a patient's body to an unintended orientation. For example, it is possible to reduce occurrence of a situation where the distal end of the shaft having an unintendedly changed orientation is caught when pulling the shaft out of the patient's body.

### EFFECTS OF THE INVENTION

The surgical tool of the present disclosure achieves an effect that unintended movement of the surgical tool can be reduced and cleaning can be facilitated by providing the one or more fixing portions each configured to move in the direction toward the gap between the driven portion and the main body and to be abuttable with the driven portion and with the main body.

### BRIEF DESCRIPTION OF THE DRAWINGS

FIG. 1 is a view showing one embodiment of a surgical tool according to the present disclosure.
FIG. 2 is a view showing a main body and a shaft of the surgical tool in FIG. 1.
FIG. 3 is a view showing the main body of the surgical tool in FIG. 1.
FIG. 4 is a view showing a fixing portion, a frame portion, and a driven portion of the surgical tool.
FIG. 5 is a view showing a fixing portion, the fixing portion, the frame portion, and the driven portion.

### EXPLANATION OF REFERENCE NUMERALS

1...surgical tool, 10...shaft, 11...joint portion, 20...main body, 25...driven portion, 37...main body-side inclined surface (first inclined surface), 41...fixing portion, 43...fixing portion inclined surface (second inclined surface), 26...driven portion inclined surface (first inclined surface), G...gap

### MODE FOR CARRYING OUT THE INVENTION

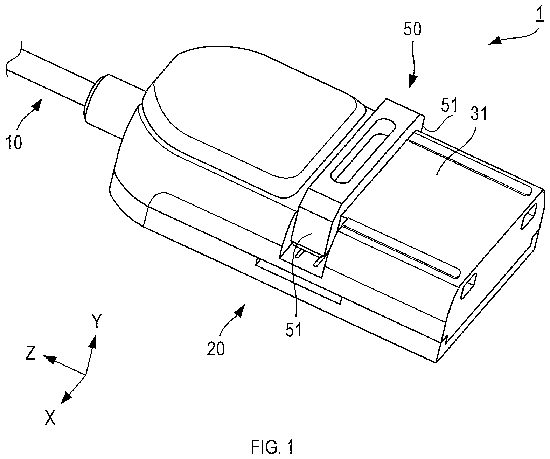

A description will be given of a surgical tool in one embodiment of the present disclosure with reference to FIG. 1 to FIG. 5. A surgical tool 1 of the present embodiment is a surgical tool to be held by a multi-degree-of-freedom manipulator having a multi-degree-of-freedom arm that is remotely operable. The surgical tool 1 may include a configuration of, for example, a forceps to be used for performing treatment of a patient, such as an endoscopic operation.

As shown in FIG. 1, the surgical tool 1 comprises a shaft 10, a main body 20, and a fastener 50.

In the present embodiment, for the purpose of simplification, a direction along which the shaft 10 extends is described as a "Z-axis", and a direction from a base of the shaft 10 toward its distal end is described as a "positive direction". Also, a direction which is orthogonal to the Z-axis and along which two or more later described driven portions 25 are aligned is described as an "X-axis", and a leftward direction relative to the positive direction of the Z-axis is described as a positive direction of the X-axis. Further, a direction orthogonal to the Z-axis and the X-axis is described as a "Y-axis", and a direction from a surface of the main body 20 on which the driven portions 25 are aligned toward an opposite surface is described as a positive direction of the Y-axis.

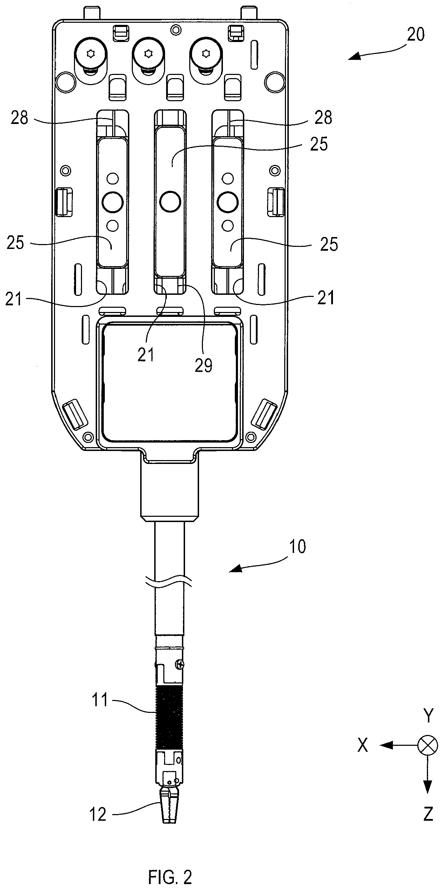

As shown in FIG. 2, the shaft 10 is a member arranged to extend from the main body 20 in the positive direction of the Z-axis, having a bar shape, and configured to be inserted into a patient's body. The shaft 10 has a columnar or cylindrical configuration.

In an end portion of the shaft 10 in the positive direction of the Z-axis, a joint portion 11 and a forceps 12 are provided.

The joint portion 11 is configured to allow changes in orientation of the forceps 12 by a driving force transmitted from the driven portions 25 described later. A specific configuration of the joint portion 11 may be any general configuration, and is not particularly limited.

The forceps 12 has a configuration of a typical forceps to be used for performing treatment of patients. In place of the forceps 12, another instrument to be used for treatment of patients may be arranged at a distal end of the shaft 10.

The main body 20 is configured to be fixed to the multi-degree-of-freedom manipulator in an attachable and detachable manner. In an end portion of the main body 20 in the positive direction of the Z-axis, the shaft 10 is arranged. Also, the main body 20 is configured to allow attachment and detachment of the fastener 50.

As shown in FIG. 2, the main body 20 comprises two or more (three in the present embodiment) driven side slits 21 (hereinafter simply referred to as a "driven side slit 21") and two or more (three in the present embodiment) driven portions 25 (hereinafter, simply referred to as a "driven portion 25").

The driven side slit 21 is an elongated hole provided in an end surface of two surfaces of the main body 20 that are perpendicular to a Y-axis direction, the end surface being located on a negative direction side of the Y-axis. In other words, the driven side slit 21 is an elongated hole provided in an attachment/detachment surface of the main body 20 to/from the multi-degree-of-freedom manipulator. Also, the driven side slit 21 is configured to extend along the Z-axis.

The three driven side slits 21 are arranged aligned at equal intervals along an X-axis direction. The number of the driven side slits 21 may be determined based on movement (movement based on required specifications) of the joint portion 11 and/or the forceps 12, for example. In accordance with required specifications, the number of the driven side slits 21 may be more than three, or may be less than three.

The driven portion 25 is configured to receive a driving force transmitted from the multi-degree-of-freedom manipulator and transmit the driving force to the joint portion 11 and the forceps 12. Also, the driven portion 25 is configured to be attachable to and detachable from the multi-degree-of-freedom manipulator.

The driven portion 25 is arranged in each of the three driven side slits 21, and the driven portion 25 is arranged so as to be movable in the driven side slit 21 along a Z-axis direction. Among the three driven portions 25, the two driven portions 25 arranged at a first end and a second end in the X-axis direction are configured to transmit a driving force to the joint portion 11. Specifically, a wire 28 to transmit the driving force is disposed at each of the two driven portions 25. Among the three driven portions 25, the driven portion 25 that is arranged centrally is configured to transmit a driving force to the forceps 12. Specifically, a rod 29 to transmit the driving force to the forceps 12 is disposed at the driven portion 25 arranged centrally.

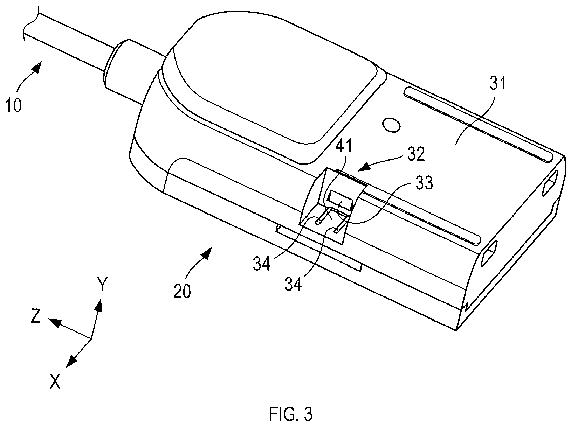

As shown in FIG. 3, a housing 31 forming an outline of the main body 20 comprises a cutout portion 32. The cutout portion 32 enables attachment and detachment between the main body 20 and the fastener 50. In FIG. 3, only one cutout portion 32 is shown. Actually, the cutout portion 32 is provided at each end portion of the housing 31 along the X-axis direction. That is, there are provided two cutout portions 32.

The cutout portion 32 is formed in an area of the housing 31, in which the driven portion 25 relatively moves with respect to the main body 20. The cutout portion 32 is formed in a central area of the housing 31 in the Z-axis direction.

The cutout portion 32 provided to the housing 31 on a positive direction side of the X-axis (the cutout portion 32 shown in FIG. 3) has a recessed shape opening in the positive direction of the X-axis and in the positive direction of the Y-axis. Although not shown in the drawings, the cutout portion 32 provided to the housing 31 on a negative direction side of the X-axis has a recessed shape opening in the negative direction of the X-axis and in the positive direction of the Y-axis.

A side surface of the cutout portion 32, specifically a surface extending along a Y-Z plane comprises an engagement recess 33 configured to be engaged with/disengaged from an engagement projection 52 of the fastener 50 described later. A bottom surface of the cutout portion 32, specifically a surface extending along a Z-X plane, comprises a fixing portion 41. In other words, the fixing portion 41 is arranged in a position to face the driven portion 25 configured to transmit the driving force to the joint portion 11. The fixing portion 41 has a configuration that is defined by two slits 34 extending along the X-axis.

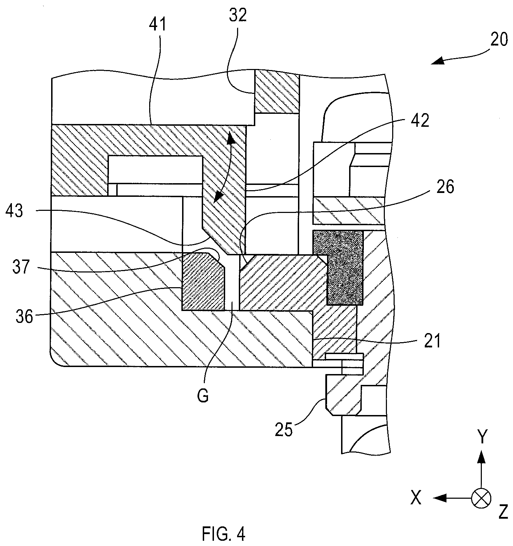

As shown in FIG. 4, the fixing portion 41 has a cantilever configuration extending from the end portion of the housing 31 toward a central portion (from a positive side toward a negative side of the X-axis in FIG. 4). A centrally located end of the fixing portion 41 comprises a protrusion 42 extending toward the driven portion 25 configured to transmit the driving force to the joint portion 11. In other words, the protrusion 42 extends from the fixing portion 41 in the negative direction of the Y-axis.

An end portion of the protrusion 42 on a side of the driven portion 25 configured to transmit the driving force to the joint portion 11 comprises a fixing portion inclined surface (corresponding to a second inclined surface) 43. The fixing portion inclined surface 43 is an inclined surface provided so as to face a main body-side inclined surface 37. Specifically, the inclined surface is such that the thickness of the protrusion 42 becomes smaller in the negative direction of the Y-axis. The fixing portion inclined surface 43 may be an inclined surface provided so as to face a driven portion inclined surface 26.

The main body 20 comprises a frame portion (corresponding to a "main body") 36 that holds the driven portion 25 to be movable in the driven side slit 21. A gap G is provided between the frame portion 36 and the driven portion 25 configured to transmit the driving force to the joint portion 11. The gap G is a space formed between a part of the frame portion 36 extending in the Z-axis direction, and the driven portion 25 configured to transmit the driving force to the joint portion 11. Due to the presence of the gap G, the driven portion 25 configured to transmit the driving force to the joint portion 11 is relatively movable with respect to the main body 20.

A main body-side inclined surface (corresponding to a "first inclined surface") 37 is provided in an area of the frame portion 36, the area facing the gap G and also facing the protrusion 42. The main body-side inclined surface 37 is an inclined surface provided so as to face the fixing portion inclined surface 43. The main body-side inclined surface 37 is also an inclined surface so as to face the driven portion inclined surface 26 described later. Specifically, the main body-side inclined surface 37 is a surface inclined in a direction of separating from the protrusion 42 toward the driven portion 25 configured to transmit the driving force to the joint portion 11.

The driven portion inclined surface (corresponding to the first inclined surface) 26 is provided in an area of the driven portion 25, the area facing the gap G and also facing the protrusion 42. The driven portion inclined surface 26 is an inclined surface so as to face the fixing portion inclined surface 43. Also, the driven portion inclined surface 26 is an inclined surface so as to face the main body-side inclined surface 37. Specifically, the driven portion inclined surface 26 is a surface inclined in a direction of separating from the protrusion 42 toward the gap G.

Although the present embodiment describes an example in which the main body-side inclined surface 37 and the driven portion inclined surface 26 are provided, only the main body-side inclined surface 37 may be provided, or only the driven portion inclined surface 26 may be provided.

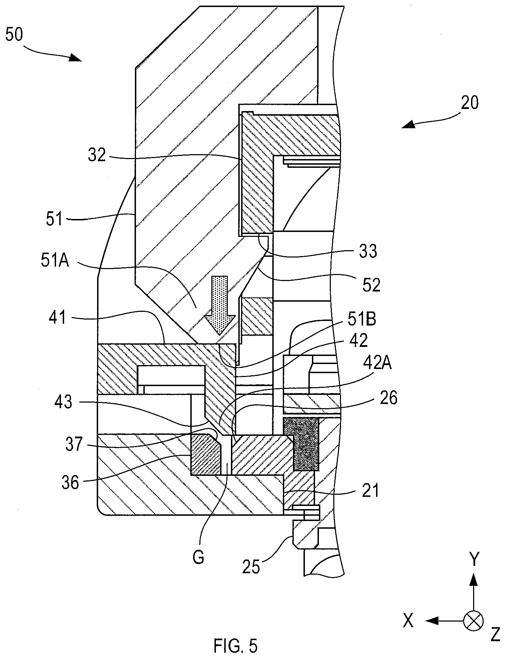

As shown in FIG. 1, the fastener 50 has a U-shaped configuration attachable to and detachable from the main body 20. The fastener 50 comprises two arm portions 51 each to be inserted into the cutout portion 32 of the main body 20. As shown in FIG. 5, the arm portion 51 comprises the engagement projection 52 to engage with the engagement recess 33.

A distal end 51A of the arm portion 51 comprises a surface 51B configured to abut the fixing portion 41 when the fastener 50 is attached to the main body 20. Specifically, the surface 51B is a surface to abut the fixing portion 41 when the engagement projection 52 of the fastener 50 and the engagement recess 33 of the main body 20 engage with each other.

Also, the arm portion 51 is configured to press down the protrusion 42 in the negative direction of the Y-axis when abutting the fixing portion 41. Specifically, a dimension from the distal end 51A of the arm portion 51 to the engagement projection 52 has a value such that the protrusion 42 is pressed down in the negative direction of the Y-axis when the engagement projection 52 of the fastener 50 and the engagement recess 33 of the main body 20 engage with each other.

Next, a description will be given of an operation of the surgical tool 1 that is configured as described above. When the surgical tool 1 is being used for treatment of a patient, the fastener 50 is detached from the main body 20 (see FIG. 3, although illustration of the fastener 50 is omitted). In this state, relative movement of the driven portion 25 with respect to the main body 20 is possible. That is, transmission of the driving force to the joint portion 11 and the forceps 12 of the surgical tool 1 is possible.

There may occur a need to restrict movement of the joint portion 11 of the surgical tool 1, for example, when the multi-degree-of-freedom manipulator with the surgical tool 1 attached makes an emergency stop. Then, the fastener 50 is attached to the main body 20, as shown in FIG. 1.

Specifically, as shown in FIG. 5, the arm portion 51 of the fastener 50 is inserted into the cutout portion 32 of the main body 20. The arm portion 51 is inserted until the engagement projection 52 engages with the engagement recess 33 of the cutout portion 32.

When the arm portion 51 is inserted, the surface 51B of the distal end 51A of the arm portion 51 abuts the fixing portion 41. An end portion of the fixing portion 41 at which the protrusion 42 is provided is pressed down in the negative direction of the Y-axis. A distal end 42A of the protrusion 42 that is pressed down contacts the driven portion inclined surface 26 or the main body-side inclined surface 37 and is guided toward the gap G.

The distal end 42A of the protrusion 42 guided as above is pushed into the gap G. Then, the fixing portion inclined surface 43 of the protrusion 42 and the main body-side inclined surface 37 abut each other. The driven portion 25 is pressed against the main body 20 or the frame portion 36 by the protrusion 42 that is pushed into the gap G.

A friction force acting between the driven portion 25 and the main body 20 or the frame portion 36 reduces relative movement of the driven portion 25 with respect to the main body 20, or fastens the driven portion 25 to the main body 20. As a result, movement of the joint portion 11, to which the driving force is transmitted from the driven portion 25, is restricted.

According to the surgical tool 1 configured as described above, the protrusion 42 of the fixing portion 41 that has moved is pressed against the driven portion 25 and the frame portion 36 of the main body 20. The driven portion 25, against which the protrusion 42 is pressed, is also pressed against the main body 20 or the frame portion 36. In an abutting area between the protrusion 42 and the driven portion 25, and in an abutting area between the driven portion 25 and the main body 20 or the frame portion 36, a friction force as a force resisting the relative movement between the driven portion 25 and the main body 20 is generated. This facilitates reduction of unintended movement of the surgical tool 1 when moving the protrusion 42 of the fixing portion 41 toward the driven portion 25 and the frame portion 36 of the main body 20.

Also, the number of components forming the surgical tool 1 is reduced as compared with that in the configuration disclosed in Patent Document 1. Further, it is easy to achieve simplified configurations of those components. Accordingly, it is easy to reduce work required for cleaning and sterilizing the surgical tool 1, and it is easy to reduce time required for the work.

By providing the main body-side inclined surface 37 and the driven portion inclined surface 26, the protrusion 42 of the fixing portion 41 is easily guided into the gap G between the driven portion 25 and the frame portion 36. The protrusion 42 of the fixing portion 41 that has been guided into the gap G is pressed against the driven portion 25 and the main body 20 or the frame portion 36. This facilitates reduction of unintended movement of the surgical tool 1 when moving the protrusion 42 of the fixing portion 41 toward the gap G between the driven portion 25 and the main body 20.

By providing the fixing portion inclined surface 43, the protrusion 42 of the fixing portion 41 is more easily guided into the gap G between the driven portion 25 and the main body 20. This facilitates further reduction of unintended movement of the surgical tool 1 when moving the protrusion 42 of the fixing portion 41 toward the gap G between the driven portion 25 and the main body 20.

Since the fixing portion inclined surface 43 abuts the main body-side inclined surface 37, an abutting area between these surfaces can be easily secured. In the abutting area between the main body-side inclined surface 37 and the fixing portion inclined surface 43, a friction force as a force resisting the relative movement between the driven portion 25 and the main body 20 is generated. This facilitates further reduction of unintended movement of the surgical tool 1 when moving the fixing portion 41 toward the gap G between the driven portion 25 and the main body.

It is configured such that two of the three driven portions 25, which are arranged aligned with one another, each abut the protrusion 42 of the fixing portion 41, and thus it is possible to differentiate between a movement of the surgical tool 1 to be restricted and a movement of the surgical tool 1 to be not restricted. Specifically, it is configured such that the driven portion 25 related to a movement of the joint portion 11 is abuttable with the fixing portion 41, and the driven portion 25 related to a movement of the forceps 12 is not abuttable with the fixing portion 41. Thus, it is possible to differentiate between an object for restriction of movement and an object not for restriction of movement.

Since it is configured such that the driven portion 25 configured to transmit the driving force to the joint portion 11 abuts the protrusion 42 of the fixing portion 41, movement of the joint portion 11 can be restricted. More specifically, it is possible to reduce changes in orientation of the distal end of the shaft 10 that is inserted in a patient's body to an unintended orientation. For example, it is possible to reduce occurrence of a situation where the distal end of the shaft 10 having an unintendedly changed orientation is caught when pulling the shaft 10 out of the patient's body

It is to be noted that the technical scope of the present disclosure is not limited to the above described embodiment, and various modifications thereto may be made without departing from the subject matter of the present disclosure. For example, although relative movement of two of the three driven portions 25 is restricted by the fixing portion 41 in the above described embodiment, relative movement of only one of the driven portions 25 may be restricted, or relative movement of all the three driven portions 25 may be restricted.

Also, although the fixing portion inclined surface 43 is provided so as to face the main body-side inclined surface 37 in the present embodiment, the fixing portion inclined surface 43 may be provided so as to face the driven portion inclined surface 26.

Further, although the forceps 12 is arranged on the shaft 10 of the surgical tool 1 in the present embodiment, any of all instruments to be used for treatment of a patient may be arranged on the shaft 10.

## Claims

1. A surgical tool, comprising:
one or more driven portions to transmit a driving force to a distal end of a shaft to be inserted into a patient's body;
a main body holding the one or more driven portions so as to be relatively movable in respective specified directions; and
one or more fixing portions each configured to move in a direction intersecting a corresponding one of the specified directions and extending toward a corresponding one of the one or more driven portions and the main body, and to be abuttable with the corresponding one of the one or more driven portions and with the main body.

2. The surgical tool according to claim 1,
wherein each of the one or more driven portions and/or the main body comprise one or more first inclined surfaces to guide a corresponding one of the one or more fixing portions to a gap between the corresponding one of the one or more driven portions and the main body.

3. The surgical tool according to claim 2,
wherein one or more second inclined surfaces are provided to each of the one or more fixing portions in positions to face the first inclined surfaces.

4. The surgical tool according to any one of claims 1 to 3,
wherein the one or more driven portions are two or more driven portions, which are arranged aligned with each other on a plane that includes the specified directions and does not include the intersecting directions, and
wherein each of the one or more fixing portions is abuttable with a corresponding one of a part of the two or more driven portions and with the main body.

5. The surgical tool according to claim 4,
wherein the shaft comprises a joint portion allowing changes in orientation of the distal end of the shaft by the driving force transmitted from the one or more driven portions, and
wherein each of the one or more fixing portions is abuttable with the corresponding one of the one or more driven portions to transmit the driving force to the joint portion and with the main body.
